# EUROPEAN PATENT SPECIFICATION

(11) **EP 1 344 548 B1**
(45) Date of publication and mention of the grant of the patent: **06.12.2006**
(21) Application number: 03000136.6
(22) Date of filing: 03.01.2003
(51) Int. Cl.: A61M 25/00

(54) **Method of trimming a balloon of a balloon catheter**
Methode zum Modellieren eines Ballons eines Ballonkatheters
Méthode pour modeler un ballonnet d'un cathéter à ballonnet

(30) Priority: 11.03.2002 EP 02005515
(43) Date of publication of application: 17.09.2003
(73) Proprietor: Abbott Laboratories Vascular Enterprises Limited, Dublin 2 (IE)
(72) Inventor: Boelens, Johan, 72379 Hechingen (DE); Trösken, Volker, 72379 Hechingen (DE)
(74) Representative: Schmitz, Hans-Werner

(56) References cited:
- EP-A- 0 834 293
- US-A- 5 066 298

## Description

The present invention concerns a method of trimming a balloon of a balloon catheter.

Balloon catheters are used for recanalisation of blocked or narrowed blood vessels. For this purpose, a known balloon catheter comprises an expandable balloon. The balloon is folded and a stent may be crimped onto the folded balloon for introducing the balloon catheter and the stent crimped thereon into the body vessel.

Known balloon catheters, however, often cause problems in particular in case of extremely narrow vessels as the balloon of known balloon catheters often cannot sufficiently hold the crimped-upon stent while being moved through the vessels.

It is therefore an object of the present invention to provide a method of trimming a balloon of a balloon catheter that makes it possible to produce a balloon that provides for improved stent fastening characteristics.

The solution of this object is achieved by the features of claim 1.

By wrapping a thread or flat band around the folded balloon and leaving that thread on the folded balloon for a predetermined period of time, it is possible to compress spaced-apart portions of the folds tightly together what results, as a side-effect, in an extremely small profile of the balloon in its folded condition, and in a rough surface of the folded balloon resulting in a higher surface friction. This, in turn, results in the advantage that the stent crimped onto the folded balloon can temporarily be fixed much easier on the balloon, thus preventing the stent from slipping from the balloon when being moved within a body vessel.

US 5,066,298 discloses a pre-compressed angioplasty balloon catheter having a balloon portion being wrapped for storage and for minimizing its outside diameter for purpose of insertion into the body. The balloon portion of said catheter is tightly wrapped in its collapsed condition with a tape. As, however, the tape is wrapped in conventional overlapping fashion the only effect of said tape is minimizing the outer balloon diameter. There is absolutely no effect with respect to an improvement of the fastening characteristics for holding a stent crimped onto said balloon.

For a pre-mounted system (balloon and stent), the use of a round, flat or otherwise shaped thread is preferable.

Moreover, the method according to the present invention results in a balloon that can be easily pushed forward through extremely curved vessels without causing the problem that the folds open when the balloon moves through such extreme curves.

Moreover, also the conical end portions of the balloon, which are stiffer or harder than the central portion of the balloon, can be compressed more easily with the method according to the present invention.

The dependent claims contain advantageous embodiments of the method according to claim 1.

So, the folded balloon can be somewhat heated when the thread is wrapped around the folded balloon, thus increasing or accelerating the effect of the higher compression and enhancing formation of the surface structure of the balloon.

Preferred materials of the thread are polytetrafluorethylene or polyurethane. Moreover, any other material is possible and any shape of thread profile or band profile can be used.

The method according to the present invention can be effected manually or automatically by a suitable machine.

Claim 9 defines a balloon catheter that is produced according to the principles of the method according to the present invention.

Further features, advantages and effects according to the present invention will become apparent from the accompanying drawings.
Fig. 1 shows a balloon along with a tape or thread wrapped around said balloon for explaining the features of the method according to the present invention,
Fig. 2 a representation of the balloon after having removed the thread or band, and
Fig. 3 the balloon according to claim 2 with a stent being crimped thereon.

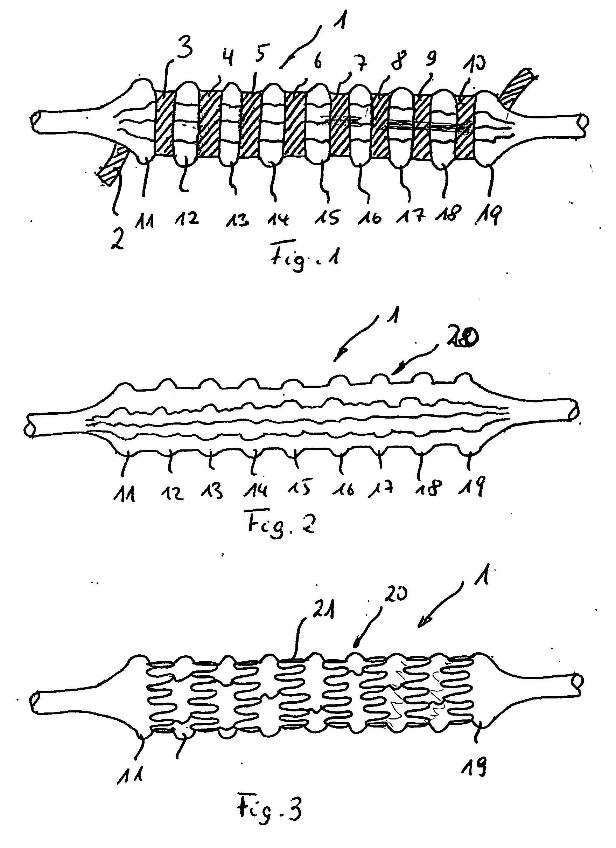

Fig. 1 shows a balloon (1) of a balloon catheter. According to the present invention the balloon can be formed and folded in a conventional manner. After having folded said balloon, a flat band (2), as shown in Fig. 1, (or alternatively a thread) is wrapped around the balloon (1) such that spaced-apart loops (3) to (10) are created. Needless to say that also a different number of loops can be formed.

As is apparent from Fig. 1 the band (2) is wrapped around balloon (1) such that loops (3) to (10) leave ring-like protrusions (11) to (17) between two respective loops (e.g. (3), (4) and (5), (6) and so on).

The band (2) is left on the balloon for a suitable period of time.

Fig. 2 shows balloon (1) with the band (2) being removed. Balloon (1) now comprises a surface structure (20) that has improved fastening characteristics as said surface structure (20) provides for a high friction so that a stent crimped on balloon (1) can be held more securely.

Such a state of balloon (1) is shown in Fig. 3 where a stent (21) is employed on surface structure (20). Fig. 3 shows that protrusions (11) to (19) secure stent (21) securely on balloon (1) as said protrusions (11) to (19) extend through the apertures in the stent structure such that the stent (21) can be prevented from slipping off balloon (1) while being moved even through a curved body vessel or a narrow stenosis.

## Claims

1. Method of trimming a balloon (1) of a balloon catheter, comprising the following method steps:
a) creating protrusions (11-19) on the balloon (1) by wrapping a thread or flat band (2) around the balloon (1) such that spaced-apart loops (3-10) of the thread or band (2) compress the balloon (1) leaving uncompressed portions of the balloon (1) therebetween constituting the protrusions (11-19);
b) leaving the thread or flat band on the balloon for a suitable period of time;
c) removing the thread or flat band from the balloon after a suitable period of time.

2. Method according to claim 1, being **characterised by** heating the balloon during method step a) and/or b).

3. Method according to claim 1 or 2, **characterised by** using polytetrafluorethylene, polyurethane or any other suitable material for the thread or flat band.

4. Method according to one of claims 1 to 3, being **characterised by** folding the balloon prior to steps a) to c).

5. Method according to one of claims 1 to 4, being **characterised by** the following method steps prior to steps a) to c):
d) introducing a tube (5) of balloon material into a balloon forming mold (1);
e) forming the tube (5) into the balloon shape;
f) taking the balloon out of the mold (1).

6. Method according to one of claims 1 to 5, being **characterised by** attaching the balloon to a catheter tubing.

7. Method according to one of claims 1 to 6 being **characterised by** effecting the method steps of claims 1 and 4 to 6 manually.

8. Method according to one of claims 1 to 6, being **characterised by** effecting the method steps of claims 1 and 4 to 6 by a machine.

9. Balloon catheter having a foldable and expandable balloon obtained by a method according to one of claims 1 to 8.

## Patentansprüche

1. Verfahren zum Modellieren eines Ballons (1) eines Ballonkatheters, umfassend die folgenden Verfahrensschritte:
a) Erzeugen von Vorsprüngen (11-19) auf dem Ballon (1) durch Wickeln eines Fadens oder Flachbandes (2) um den Ballon (1), so dass voneinander beabstandete Schlingen (3-10) des Fadens oder Flachbandes (2) den Ballon (1) zusammendrücken, wobei nicht-zusammengedrückte Bereiche des Ballons (1) dazwischen erhalten bleiben, welche die Vorsprünge (11-19) bilden;
b)Belassen des Fadens oder Flachbandes auf dem Ballon über einen geeigneten Zeitraum,
c)Entfernen des Fadens oder Flachbandes vom Ballon nach einem geeigneten Zeitraum.

2. Verfahren nach Anspruch 1, **gekennzeichnet durch** Erwärmen des Ballons während Verfahrenschritt a) und/oder b).

3. Verfahren nach Anspruch 1 oder 2, **gekennzeichnet durch** Verwendung von Polytetrafluorethylen, Polyurethan oder eines anderen geeigneten Materials für den Faden oder das Flachband.

4. Verfahren nach einem der Ansprüche 1 bis 3, **gekennzeichnet durch** Falten des Ballons vor den Schritten a) bis c).

5. Verfahren nach einem der Ansprüche 1 bis 4, **gekennzeichnet durch** die folgenden Verfahrensschritte vor den Schritten a) bis c):
d)Einführen eines Rohrs (5) aus Ballonmaterial in eine Ballon-Formgebungsform (1);
e) Formen des Rohrs (5) in die Ballonform;
f)Entnehmen des Ballons aus der Form (1).

6. Verfahren nach einem der Ansprüche 1 bis 5, **gekennzeichnet durch** Befestigen des Ballons an einem Katheterrohr.

7. Verfahren nach einem der Ansprüche 1 bis 6, **gekennzeichnet durch** manuelles Ausführen der Verfahrensschritte der Ansprüche 1 und 4 bis 6.

8. Verfahren nach einem der Ansprüche 1 bis 6, **gekennzeichnet durch** maschinelles Ausführen der Verfahrensschritte der Ansprüche 1 und 4 bis 6.

9. Ballonkatheter mit einem faltbaren und ausdehnbaren Ballon, erhalten durch ein Verfahren nach einem der Ansprüche 1 bis 8.

## Revendications

1. Procédé d'ajustement d'un ballon (1) d'un cathéter à ballon, comprenant les étapes de procédé suivantes consistant à :
a) créer des saillies (11-19) sur le ballon (1) en enroulant un fil ou une bande plate (2) autour du ballon (1) de sorte que des boucles espacées (3-10) du fil ou de la bande (2) compriment le ballon (1) en laissant des parties non comprimées du ballon (1) entre celles-ci, constituant les saillies (11-19) ;
b) laisser le fil ou la bande plate sur le ballon pendant une période de temps appropriée ;
c) enlever le fil ou la bande plate du ballon après une période de temps appropriée.

2. Procédé selon la revendication 1, étant **caractérisé par** le chauffage du ballon durant l'étape de procédé a) et/ou b).

3. Procédé selon la revendication 1 ou 2, **caractérisé par** l'utilisation de polytétrafluoroéthylène, de polyuréthane ou d'un autre matériau adapté quelconque pour le fil ou la bande plate.

4. Procédé selon l'une des revendications 1 à 3, étant **caractérisé par** le pliage du ballon avant les étapes a) à c).

5. Procédé selon l'une des revendications 1 à 4, étant **caractérisé par** les étapes de procédé suivantes avant les étapes a) à c) consistant à :
d) introduire un tube (5) de matériau de ballon dans un moule de formation de ballon (1) ;
e) former le tube (5) en forme de ballon ;
f) retirer le ballon du moule (1).

6. Procédé selon l'une des revendications 1 à 5, étant **caractérisé par** la fixation du ballon à une tubulure de cathéter.

7. Procédé selon l'une des revendications 1 à 6 étant **caractérisé par** la conduite des étapes de procédé des revendications 1 et 4 à 6 manuellement.

8. Procédé selon l'une des revendications 1 à 6, étant **caractérisé par** la conduite des étapes de procédé des revendications 1 et 4 à 6 par une machine.

9. Cathéter à ballon ayant un ballon pliable et expansible obtenu par un procédé selon l'une des revendications 1 à 8.
